(19) Europäisches Patentamt
European Patent Office
Office européen des brevets

(11) **EP 2 474 354 B1**

(12) **FASCICULE DE BREVET EUROPEEN**

(45) Date de publication et mention de la délivrance du brevet:
**11.09.2019 Bulletin 2019/37**

(51) Int Cl.:
***B01J 23/44*** *(2006.01)*     ***B01J 37/02*** *(2006.01)*

(21) Numéro de dépôt: **11290529.4**

(22) Date de dépôt: **17.11.2011**

(54) **NOUVEAU PROCÉDÉ DE PREPARATION DE CATALYSEURS À BASE DE PALLADIUM ET UTILISATION DE CES CATALYSEURS EN HYDROGÉNATION SELECTIVE**

NEUES HERSTELLUNGSVERFAHREN VON KATALYSATOREN AUF DER BASIS VON PALLADIUM, UND VERWENDUNG DIESER KATALYSATOREN BEI SELEKTIVEN HYDRIERVERFAHREN

NOVEL METHOD FOR PREPARING PALLADIUM-BASED CATALYSTS AND USE OF SAID CATALYSTS IN SELECTIVE HYDROGENATION

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **14.12.2010 FR 1004878**

(43) Date de publication de la demande:
**11.07.2012 Bulletin 2012/28**

(73) Titulaire: **IFP Energies nouvelles**
**92500 Rueil-Malmaison (FR)**

(72) Inventeur: **Fecant, Antoine**
**69530 Brignais (FR)**

(74) Mandataire: **IFP Energies nouvelles**
**Département Propriété Industrielle**
**Rond Point de l'échangeur de Solaize**
**BP3**
**69360 Solaize (FR)**

(56) Documents cités:
**EP-A1- 1 247 574**     **EP-A1- 2 075 061**
**EP-A2- 1 201 787**     **WO-A1-2005/123255**
**GB-A- 2 052 294**     **JP-A- 2002 001 119**
**JP-A- 2005 314 739**

**Description**

L'invention concerne un nouveau procédé de préparation de catalyseurs métalliques supportés dans lequel la phase métallique est déposée sous forme d'agglomérats de nanoparticules d'oxyde métallique et forme une couche de fine épaisseur à la surface du support. L'invention s'applique au domaine du raffinage et plus particulièrement au traitement des essences obtenues par vapocraquage (essence de pyrolyse) et/ou obtenues par craquage catalytique.

Les composés organiques mono-insaturés tels que par exemple l'éthylène, le propylène, sont à la source de la fabrication de polymères, de matières plastiques et d'autres produits chimiques à valeur ajoutée. Ces composés sont obtenus à partir du gaz naturel, du naphta ou du gazole qui ont été traités par des procédés de vapocraquage ou de craquage catalytique. Ces procédés sont opérés à haute température et produisent, en plus des composés mono-insaturés recherchés, des composés organiques polyinsaturés tels que l'acétylène, le propadiène et le méthylacétylène (ou propyne), le 1-2-butadiène et le 1-3-butadiène, le vinylacétylène et l'éthylacétylène, et d'autres composés polyinsaturés dont le point d'ébullition correspond à la fraction essence C5$^+$ (essences contenant des composés hydrocarbonés ayant plus de 5 atomes de carbone). Ces composés polyinsaturés sont très réactifs et conduisent à des réactions parasites dans les unités de polymérisation. Il est donc nécessaire de les éliminer avant de valoriser les coupes contenant des composés organiques mono-insaturés.

Ainsi, par exemple, la coupe C2 (essence contenant des composés hydrocarbonés ayant 2 atomes de carbone) de vapocraquage peut avoir la composition volumique moyenne suivante : 1,2% poids d'acétylène, 83,5% poids d'éthylène et 15,3% poids d'éthane. Cette coupe peut être utilisée dans une unité de polymérisation si elle respecte les spécifications concernant les concentrations en acétylène pour les unités de polymérisation, c'est à dire si la concentration en acétylène est inférieure à 2 ppm poids (ppm : partie par million). Il en est de même pour les coupes C3 et C4 ou les autres coupes où les spécifications sont également très sévères pour leurs utilisations dans les unités de polymérisation.

L'hydrogénation sélective est le principal traitement développé pour éliminer spécifiquement les composés polyinsaturés indésirables des essences de pyrolyse ou de craquage catalytique contenant principalement des mono-oléfines. Elle permet la conversion des composés polyinsaturés vers les alcènes ou aromatiques correspondants en évitant leur saturation totale et donc la formation des alcanes correspondants. Ainsi dans une coupe C2, après un traitement d'hydrogénation sélective, la teneur en acétylène sera réduite tandis que la teneur en éthylène sera augmentée et la teneur en éthane pratiquement inchangée.

Les catalyseurs d'hydrogénation sélective sont généralement à base de métaux du groupe VIII du tableau périodique des éléments, de préférence le palladium ou le nickel. La phase active des catalyseurs se présente sous la forme de petites particules métalliques déposées sur un support qui peut être un oxyde réfractaire sous forme de billes, d'extrudés, de trilobes ou sous des formes présentant d'autres géométries. La teneur en métal, la taille des particules de métal et la répartition de la phase active dans le support font partie des critères qui ont une importance sur l'activité et la sélectivité de ces catalyseurs.

Les particules métalliques supportées peuvent posséder une taille moyenne comprise entre 1 et 5 nm. Cette taille est adaptée aux exigences des réactions d'hydrogénation sélective. En effet, la vitesse de réaction d'hydrogénation de molécules polyinsaturées telles que les dioléfines ou les acétyléniques dépend de la taille des particules métalliques. Ce résultat est généralement décrit sous le terme « sensibilité à la structure ». Un optimum est généralement observé pour une taille de l'ordre de 3 à 4 nm, cette valeur pouvant varier en fonction notamment de la masse moléculaire des réactifs (M. Boudart, W.C. Cheng, J. Catal. 106, 1987, 134, S. Hub, L. Hilaire, R. Touroude, Appl. Catal. 36 1992, 307).

La répartition de la phase active au sein des catalyseurs d'hydrogénation sélective joue un rôle décisif en ce qui concerne leur activité et sélectivité. Par imprégnation d'une solution de précurseurs de palladium ioniques sur un support de type oxyde réfractaire, les espèces actives diffusent au coeur du support. Cette répartition de la phase métallique au coeur du support est préjudiciable, particulièrement dans le cas de réactions contrôlées par la diffusion des réactifs, comme c'est le cas pour l'hydrogénation sélective. Ceci implique une faible activité et une faible sélectivité des catalyseurs puisque des réactions secondaires indésirables ont alors lieu. Pour qu'un catalyseur d'hydrogénation ait de bonnes propriétés catalytiques, il est préférable que les particules métalliques soient déposées à la surface du support sous forme d'une couche de faible épaisseur. Lorsque les particules sont réparties de telle manière, on parle de dépôt en croute et/ou de catalyseur à coquille. Plus l'épaisseur de cette couche est fine, plus les problèmes de transfert de matière intragranulaire qui peuvent conduire à des défauts d'activité et à une perte de sélectivité sont évités.

L'utilisation de solutions colloïdales de particules métalliques ou de particules d'oxyde métallique permet d'obtenir des catalyseurs dont la taille et la répartition des particules métalliques répondent en partie aux critères décrits ci-dessus. La demande de brevet EP0979673 décrit la préparation d'un catalyseur d'hydrogénation sélective par imprégnation d'une suspension colloïdale d'oxyde métallique en phase aqueuse sur un support.

L'utilisation de solutions colloïdales de nanoparticules métalliques ou de nanoparticules d'oxyde métallique dans des procédés de fabrication de catalyseurs supportés s'est développée ces dernières années. Cependant, cette technique présente de nombreux inconvénients. En effet, les solutions colloïdales ne sont pas stables thermiquement. Les nanoparticules ont tendance à coalescer et à s'agglomérer provoquant leur précipitation en solution. Cette agrégation entraîne habituellement une perte d'activité des nanoparticules. La stabilisation des nanoparticules et donc le maintien de leur caractère finement dispersé en solution est une étape fondamentale lors de leur synthèse et lors de leur dépôt sur le support. La stabilisation des nanoparticules est obtenue par l'utilisation d'agents stabilisants. Ces agents permettent de contrôler la taille des nanoparticules lors de leur croissance. Ils permettent d'obtenir des nanoparticules dispersées en solution et de taille homogène. Ils évitent ainsi qu'elles ne s'agrègent et qu'elles ne précipitent en solution aqueuse. L'utilisation de ces stabilisants est décrite dans les documents suivants. La demande de brevet WO 00/29332 décrit un procédé pour obtenir une solution colloïdale stable de nanoparticules d'oxyde métallique présentant une taille de 0,5 à 5 nm. Les travaux de Klasovsky et al. (Topic in Catalysis, vol 52, p. 412-423, 2009) étudient la préparation de catalyseurs d'hydrogénation sélective à base de palladium par l'imprégnation d'une solution colloïdale de nanoparticules d'oxyde de palladium stabilisée à l'aide d'un polymère (polyvinylpyrrolidone - PVP) issues de l'hydrolyse de $H_2PdCl_4$ en solution avec différentes bases organiques ou minérales. Enfin, il est également connu du brevet EP0653243 de préparer un catalyseur à base de métal de transition en dissolvant un précurseur dudit métal en solution auquel est ajouté un polymère organique permettant une répartition sélective de la phase métallique dans les macropores du solide après mise en forme du catalyseur. Dans les procédés décrits ci-dessus, les nanoparticules métalliques stabilisées et dispersées en solution en présence d'agents stabilisants sont ensuite réduites par un agent réducteur avant d'être déposées sur le support.

De manière totalement surprenante et contrairement aux procédés de l'art antérieur, la demanderesse a constaté que des catalyseurs supportés peuvent être préparés à partir de nanoparticules d'oxyde métallique qui forment des agglomérats en présence d'au moins un agent agglomérant. L'invention présente les avantages suivants:

- la préparation des catalyseurs supportés avec une solution colloïdale d'agglomérats de nanoparticules d'oxyde métallique est simplifiée car elle permet de s'affranchir du problème de stabilité des nanoparticules,
- les catalyseurs obtenus selon le procédé de l'invention ont une phase métallique qui est concentrée à la surface du support. On obtient des catalyseurs présentant une phase active déposée en croûte.
- les catalyseurs obtenus selon le procédé de l'invention présentent des propriétés catalytiques améliorées par rapport aux catalyseurs connus.

Dans la suite, les groupes d'éléments chimiques sont donnés selon la classification CAS (CRC Handbook of Chemistry and Physics, éditeur CRC press, rédacteur en chef D.R. Lide , 81ème édition, 2000-2001). Par exemple, le groupe VIII selon la classification CAS correspond aux métaux des colonnes 8, 9 et 10 selon la nouvelle classification IUPAC.

Par "nanoparticules d'oxyde métallique" ou "nanoparticules d'oxyde de métal" ou "nanoparticules", on entend au sens de la présente invention, des particules polycristallines d'un oxyde de métal. La taille d'une nanoparticule est comprise entre 1 et 10 nm. De manière préférée, la taille d'une nanoparticule est comprise entre 1 et 5 nm.

Par "agglomérat", on entend au sens de la présente invention, un ensemble de nanoparticules d'oxyde de métal qui se regroupent par l'effet d'interactions faibles de type Van der Waals ou électrostatiques pour former un agglomérat ou un agrégat. Les nanoparticules formant les agglomérats ne sont pas liées entre elles chimiquement par des liaisons de covalence. La taille des agglomérats est comprise entre 20 et 200 nm, de préférence entre 20 et 150 nm, de manière encore plus préférée entre 20 et 100 nm.

Par "suspension aqueuse colloïdale" ou "suspension colloïdale aqueuse", on entend au sens de la présente invention, une solution aqueuse contenant des nanoparticules d'oxyde métallique et/ou des agglomérats de nanoparticules d'oxyde métallique qui sont en suspension dans la solution aqueuse et qui forment un mélange homogène sans qu'ils ne précipitent ou ne sédimentent.

Par "V.V.H." on entend au sens de la présente invention, la vitesse volumétrique horaire définit comme le rapport entre le débit volumique de la charge à traité et le volume de catalyseur chargé dans le réacteur. La vitesse volumétrique horaire s'exprime en $h^{-1}$.

Par "point de charge nulle", on entend au sens de la présente invention, le pH de la solution aqueuse dans laquelle le solide existe sous un potentiel électrique neutre.

Un objet de l'invention concerne un procédé de préparation d'un catalyseur dans lequel on prépare en phase aqueuse une suspension colloïdale d'agglomérats de nanoparticules d'oxyde métallique, puis on dépose cette suspension sur un support poreux, on sèche le précurseur de catalyseur obtenu, et éventuellement on calcine et on réduit le précurseur au moyen de tout composé réducteur. Dans le procédé de préparation selon l'invention, la suspension colloïdale d'agglomérats de nanoparticules d'oxyde métallique est préparée en solution aqueuse, en absence d'agent réducteur et en absence de solvant organique. L'étape de réduction de l'oxyde métallique en métal s'effectue après le dépôt de ladite suspension colloïdale d'agglomérats sur le support.

Contrairement aux techniques de l'art antérieur où l'on cherche à déposer la phase active sous forme de nanoparticules stabilisées et dispersées en solution, la demanderesse a trouvé que la phase métallique peut être déposée sur un support poreux sous une forme d'agglomérats de nanoparticules d'oxyde métallique. De façon surprenante, le dépôt de la phase métallique sous forme d'agglomérats améliore les performances catalytiques du catalyseur. L'utilisation de ces agglomérats permet de déposer la phase métallique du catalyseur à la surface du support, sous forme d'une très fine couche. De part la taille des agglomérats de nanoparticules d'oxyde métallique, ceux-ci voit leur diffusion au coeur du support fortement limitée. Ils restent donc à la surface du support et forment une fine couche de phase active à sa surface. L'épaisseur de la couche va dépendre de la quantité de métaux présents sur le catalyseur. Le dépôt de la phase métallique sous forme d'agglomérats de nanoparticules d'oxyde métallique permet de concentrer les nanoparticules à la surface du catalyseur. Lorsque le métal est le palladium, au moins 80% du palladium supporté est compris à l'intérieur d'une couche de surface dont l'épaisseur n'excède pas 80 $\mu$m.

Le procédé de préparation d'un catalyseur selon l'invention comprend plusieurs étapes:

(a) on prépare une suspension aqueuse colloïdale d'agglomérats de nanoparticules d'oxyde métallique,
(b) on dépose ladite suspension aqueuse colloïdale d'agglomérats obtenue à l'étape (a) sur un support poreux,
(c) on sèche le produit obtenu à l'étape (b),

suivit éventuellement d'au moins un des étapes suivantes:

(d) on calcine le produit obtenu à l'étape (c),
(e) on réduit le produit obtenu à l'étape (c) ou l'étape (d).

La suspension colloïdale d'agglomérats de nanoparticules d'oxyde métallique est obtenue par hydrolyse en solution aqueuse d'au moins un sel d'un précurseur métallique de l'oxyde métallique en présence d'au moins un agent agglomérant. L'hydrolyse du sel d'un précurseur métallique en pH acide ou pH basique conduit à la formation de nanoparticules d'oxyde métallique ou d'hydroxyde métallique en suspension. En présence de l'agent agglomérant, les nanoparticules d'oxyde métallique se regroupent sans précipiter et forment des agglomérats dont la taille est comprise entre 20 et 200 nm.

Selon une variante préférée du procédé, l'hydrolyse peut être par exemple réalisée par neutralisation avec au moins une base minérale telle que l'ammoniaque ou les hydroxydes d'alcalins. De manière préférée la base minérale est choisie parmi le groupe formé par l'ammoniaque, la soude et la potasse.

Lorsque l'hydrolyse s'effectue en pH acide et par neutralisation avec une base minérale, la solution de base minérale est versée dans la solution de sel de précurseur de métal.

Lorsque l'hydrolyse s'effectue en pH basique et par neutralisation avec une base minérale, la solution de sel de précurseur métallique est versée dans la solution de la base minérale.

Le métal du sel du précurseur métallique est un métal du groupe VIII choisi dans le groupe constitué par le nickel et le palladium. De façon très préférée, le métal est le palladium.

Le sel du précurseur du nickel ou du palladium peut être un sel d'un précurseur considéré présentant un degré d'oxydation du métal supérieur à 0 et soluble en solution aqueuse. Le sel du précurseur peut être choisi dans le groupe constitué par un halogénure, un oxyde, un hydroxyde, un nitrate et un sulfate du métal.

De manière plus préférée, le sel du précurseur métallique peut être choisi dans le groupe constitué par le chlorure de palladium, le bromure de palladium, l'iodure de palladium, l'hexachloropalladate de potassium, le tétrabromopalladate de potassium, le tétrachloropalladate de potassium, l'hexachloropalladate de sodium, le tétrachloropalladate de sodium, le nitrate de palladium, le sulfate de palladium, l'acétate de palladium, le chlorure de nickel, le bromure de nickel, l'iodure de nickel, le nitrate de nickel, le sulfate de nickel, l'acétate de nickel.

De manière très préférée, le sel précurseur du palladium est sélectionné dans le groupe constitué par le chlorure de palladium, le nitrate de palladium et le sulfate de palladium. De manière très préférée, le sel précurseur du palladium est le nitrate de palladium.

De manière très préférée, le sel précurseur du nickel est sélectionné dans le groupe constitué par le chlorure de nickel, le nitrate de nickel et le sulfate de nickel.

La concentration de la solution aqueuse du sel du précurseur du nickel ou du palladium est ajustée selon la teneur massique en métal voulue sur le catalyseur. La teneur massique en nickel ou en palladium par rapport à la masse de support est comprise entre 0,01 et 20% poids, de préférence entre 0,05 et 10% poids.

Lorsque le métal du groupe VIII est le palladium, la concentration de la solution aqueuse du sel de précurseur de palladium est ajustée selon la teneur massique en palladium voulue sur le catalyseur. La teneur massique en palladium par rapport à la masse de support est comprise entre 0,01 et 2% poids, de préférence entre 0,05 et 1% poids.

Lorsque le métal du groupe VIII est le nickel, la concentration de la solution aqueuse du sel de précurseur de palladium est ajustée selon la teneur massique en nickel voulue sur le catalyseur. La teneur massique en palladium par rapport à la masse de support est comprise entre 1 et 20% poids, de préférence entre 2 et 15% poids.

En solution aqueuse, la surface des nanoparticules d'oxyde métallique est chargée électriquement. La charge électrique de la surface de ces nanoparticules dépend du pH de la solution aqueuse et du point de charge nulle de l'oxyde métallique. Lorsque le pH de la solution aqueuse est supérieur au point de charge nulle de l'oxyde métallique, la surface des nanoparticules est chargée négativement. En revanche, lorsque le pH de la solution aqueuse est inférieur au point de charge nulle de l'oxyde métallique, la surface des nanoparticules est chargée positivement. L'agent agglomérant est choisi en fonction du pH de la solution aqueuse colloïdale. Il est de charge électrique opposée à la charge de surface des nanoparticules. Ainsi, lorsque le pH de la solution colloïdale est supérieur au point de charge nulle de l'oxyde métallique, l'agent agglomérant choisi est chargé positivement. On parlera dans la suite du texte d'agent agglomérant cationique ou d'agent agglomérant anionique. Plusieurs molécules d'agent agglomérant créent des liaisons électrostatiques avec les charges de la surface des nanoparticules qui sont de signe électrique opposé. Les molécules d'agent agglomérant remplacent les contre-ions qui stabilisaient les nanoparticules en solution. L'hydrophobie du groupement R de l'agent agglomérant entraîne le regroupement des molécules d'agent agglomérant entre elles et donc un regroupement des nanoparticules piégées par ledit agent agglomérant. Il se forme ainsi des agglomérats de nanoparticules d'oxyde métallique de taille comprise entre 20 et 200 nm.

L'ajout de l'agent agglomérant peut s'effectuer simultanément à la dissolution du sel du précurseur métallique ou après que le sel soit complément dissous. Il peut également être ajouté à la solution contenant la base minérale.

Selon l'invention, l'agent agglomérant est un agent agglomérant anionique ajouté à la solution de précurseur métallique sous forme d'un sel dont le contre-ion est le sodium. L'agent agglomérant anionique sous forme de sel est choisi dans le groupe formé par le dodécylsulfate de sodium et le dodécylbenzènesulfonate de sodium.

L'agent agglomérant anionique peut être synthétisé par toutes techniques de synthèse organique bien connues de l'homme du métier.

Le rapport molaire entre l'agent agglomérant, anionique, et le sel du précurseur métallique est compris entre 0,01 et 50, de manière très préférée entre 0,02 et 10.

La préparation de ladite suspension colloïdale s'effectue à une température comprise entre 10° et 50°C, de préférence entre 15° et 30°C et dans des conditions normales de pression (pression atmosphérique).

Ladite suspension colloïdale d'agglomérats ainsi préparée est telle qu'au moins 20% en nombre des nanoparticules d'oxyde de métal se trouvent sous forme d'agglomérats d'une taille 10 à 200 nm, de manière préférée au moins 30% en nombre des nanoparticules d'oxyde de métal se trouvent sous forme d'agglomérats d'une taille 10 à 200 nm et de manière très préférées au moins 50% en nombre des nanoparticules d'oxyde de métal se trouvent sous forme d'agglomérats d'une taille 10 à 200 nm. De manière préférée, la suspension colloïdale d'agglomérat ainsi préparée est telle qu'entre 20% et 95 % en nombre des nanoparticules d'oxyde de métal se trouvent sous forme d'agglomérats, de manière préférée qu'entre 30% et 90% en nombre des nanoparticules d'oxyde de métal se trouvent sous forme d'agglomérats et de manière très préférées qu'entre 50% et 90% en nombre des nanoparticules d'oxyde de métal se trouvent sous forme d'agglomérats.

La taille des agglomérats peut être mesurée par microscopie électronique à transmission (MET). La répartition en nombre des nanoparticules d'oxyde de métal sous forme d'agglomérats et sous forme de particules isolées est obtenue par un dénombrement sur les clichés de MET. Le dénombrement est effectué à partir d'un nombre de clichés important pour dénombrer 200 à 1000 particules. L'ensemble des clichés est ensuite traité pour décompte des particules présentes sous forme d'agglomérats et des particules isolées. Le rapport de ces deux valeurs donnant la proportion en nombre de nanoparticules d'oxyde métallique se trouvant sous forme d'agglomérats.

Ladite suspension colloïdale d'agglomérats est déposée sur un support poreux. Le dépôt de cette suspension peut s'effectuer par toutes les techniques connues de l'homme du métier. L'imprégnation du support peut être réalisée par imprégnation à sec, en excès ou en défaut, en mode statique ou dynamique. L'imprégnation à sec est préférée. L'imprégnation peut être réalisée en une ou plusieurs imprégnations successives.

L'imprégnation est de préférence réalisée dans des conditions où le volume de solution correspond environ au volume poreux du support. D'une façon préférée, la suspension colloïdale d'agglomérats est versée sur le support poreux. Ce processus peut être réalisé soit de façon discontinue, c'est à dire que l'étape de préparation de la suspension colloïdale précède l'étape d'imprégnation sur le support et que l'essentielle de la suspension colloïdale est envoyée en une seule fois vers l'étape d'imprégnation, soit en continu, c'est-à-dire que le produit obtenu dans la première étape est envoyé aussitôt à la seconde étape.

Le support peut comprendre au moins un oxyde réfractaire choisi dans le groupe constitué par les oxydes de magnésium, d'aluminium, de silicium, de zirconium, de thorium, ou de titane, pris seul ou en mélange entre eux ou avec d'autres oxydes de la classification périodique, tel que la silice-alumine. De préférence, le support est un oxyde d'aluminium (alumine) ou de silice. Le support peut également être un charbon, un silico-aluminate, une argile ou tout autre composé connu pour être utilisé comme support. De préférence, le support présente une surface BET comprise entre 5 et 300 m$^2$/g, de façon encore plus avantageuse entre 10 et 200m$^2$/g. La surface spécifique BET est mesurée par physisorption à l'azote. Le volume poreux total du support est généralement compris entre 0,1 et 1,5 cm$^3$/g. Le volume poreux total est mesuré par porosimétrie au mercure selon la norme ASTM D4284-92 avec un angle de mouillage de 140°, par exemple au moyen d'un appareil modèle Autopore III de la marque Microméritics.

Le support peut être mis en forme de billes, d'extrudés, de trilobes de pastilles ou d'agglomérats irréguliers et non sphériques dont la forme spécifique peut résulter d'une étape de concassage ou de monolithe. De manière avantageuse, ledit support se présente sous forme de billes ou d'extrudés.

Le catalyseur imprégné est ensuite séché afin d'éliminer toute ou une partie de l'eau introduite lors de l'imprégnation, de préférence à une température comprise entre 50 et 250°C, de manière plus préférée entre 70°C et 200°C. Le séchage est effectué sous air, ou sous atmosphère inerte (azote par exemple).

Éventuellement, le catalyseur séché est lavé à une température comprise entre 5°C et 100°C, de préférence entre 15°C et 50°C, de manière préférée le liquide utilisé pour cette étape de lavage est de l'eau ou de l'éthanol. Puis, une deuxième étape de séchage est effectuée telle que décrite précédemment.

Le catalyseur est ensuite calciné sous flux gazeux, de manière préférée sous air, sous hydrogène, sous azote ou un mélange d'au moins deux de ces gaz à une V.V.H. comprise entre 100 et 5000 $h^{-1}$. La température de calcination est généralement comprise entre 150°C et 900°C, de préférence comprise entre environ 200°C et environ 500°C. La durée de calcination est généralement comprise entre 0,5 heures et 24 heures, de manière préférée de 1 heure à 12 heures. L'étape de calcination peut être opérée par palier de température, jusqu'à la température de consigne maximale définie.

Le catalyseur est généralement réduit. Cette étape est de préférence réalisée en présence d'un gaz réducteur *in situ,* c'est-à-dire dans le réacteur où est réalisée la transformation catalytique, comprenant entre 25 vol% et 100 vol% d'hydrogène, de préférence 100 vol% d'hydrogène. Dans ce cas-là, le gaz réducteur est l'hydrogène. De manière préférée, cette étape est effectuée à une température comprise entre 50°C et 400 °C, de manière encore plus préférée entre 80°C et 160°C.

A l'issue des étapes de préparation du catalyseur, la teneur massique en nickel ou en palladium par rapport à la masse de support est comprise entre 0,01 et 20% poids, de préférence entre 0,05 et 10% poids. Lorsque le métal du groupe VIII est le palladium, la teneur massique en palladium par rapport à la masse de support est comprise entre 0,01 et 2% poids, de préférence entre 0,05 et 1% poids.

Selon une variante de préparation du catalyseur, le catalyseur est préparé en plusieurs imprégnations. Pour les catalyseurs préparés en deux imprégnations, les enchaînements peuvent être les suivants :

- Imprégnation n°1 - Séchage - Imprégnation n°2 - Séchage - Calcination.
- Imprégnation n°1 - Séchage - Calcination - Imprégnation n°2 - Séchage - Calcination.

Le catalyseur obtenu selon le procédé de l'invention peut être utilisé dans des réactions faisant intervenir des coupures ou des formations de liaisons carbone-carbone. Le catalyseur obtenu selon le procédé de l'invention permet une hydrogénation sélective des composés comportant des fonctions acétyléniques, diéniques, oléfiniques, aromatiques, cétones, aldéhydes, acides et/ou nitro. Le catalyseur obtenu selon le procédé de l'invention peut aussi être utilisé pour l'hydrogénation du monoxyde de carbone en méthanol ou en alcool en C1-C6 après une augmentation de la chaine alkyle. Il peut aussi être utilisé pour la formation du diméthyl-éther par condensation de deux molécules de méthanol. Enfin, le catalyseur obtenu selon le procédé de l'invention peut aussi être utilisé pour des réactions d'isomérisation ou d'hydro-isomérisation, d'hydrogénolyse de composés hydrocarbonés. De manière préférée, le catalyseur obtenu selon le procédé de l'invention peut être utilisé pour une réaction d'hydrogénation sélective des composés comprenant au moins une fonction diénique et/ou acétylénique.

Les conditions opératoires utilisées pour ces réactions sont les suivantes : une température comprise entre 0 et 500°C, de préférence entre 25 et 350°C, une pression comprise entre 0,1 et 20 MPa, de préférence entre 0,1 et 10 MPa, une vitesse volumique horaire (V.V.H.) comprise entre 0,1 et 50 $h^{-1}$, de préférence entre 0,5 et 30 $h^{-1}$ pour une charge liquide; et entre 500 et 30 000 $h^{-1}$, de préférence entre 500 et 15 000 $h^{-1}$ pour une charge gazeuse. Lorsque de l'hydrogène est présent, le rapport volumique d'hydrogène sur la charge est compris entre 1 et 500 litres par litre, de préférence entre 10 et 250 litres par litre.

La mise en oeuvre du catalyseur préparé selon le procédé de l'invention et les conditions de son utilisation sont à adapter par l'utilisateur à la réaction et à la technologie utilisées. En général, la mise en oeuvre est réalisée par injection de la charge hydrocarbonée à traiter et de l'hydrogène dans au moins un réacteur contenant ledit catalyseur, le réacteur étant à lit fixe, à lit mobile ou à lit bouillonnant, de manière préférée dans un réacteur à lit fixe. La totalité de ladite charge est préférentiellement injectée à l'entrée du réacteur où se produit la réaction d'hydrogénation sélective. Toutefois, il peut être avantageux, dans certains cas, d'injecter une fraction ou la totalité de ladite charge entre deux lits catalytiques consécutifs placés dans ledit réacteur. Ce mode de réalisation permet notamment de continuer à maintenir le réacteur opérationnel même lorsque l'entrée dudit réacteur se trouve bouchée par dépôts de polymères, de particules ou de gommes présentes dans ladite charge.

Le procédé d'hydrogénation sélective comprend la mise en contact d'une charge hydrocarbonée comprenant au moins un composé polyinsaturé avec le catalyseur obtenu selon le procédé décrit ci-dessus.

Ladite charge hydrocarbonée comprend au moins un composé polyinsaturé est choisie dans le groupe constitué par les coupes issues du craquage catalytique, par les coupes C2 de vapocraquage, les coupes C3 de vapocraquage, les coupes C4 de vapocraquage, les coupes C5 de vapocraquage et les essences de vapocraquage appelée aussi essences de pyrolyse. La coupe C5 de vapocraquage et les essences de pyrolyse sont appelées dans la suite de la description coupe C5+.

L'hydrogénation sélective des coupes C2, C3, C4 et C5+ peut être réalisée en phase gaz ou en phase liquide, de préférence en phase liquide. En effet, une réaction en phase liquide permet d'abaisser le coût énergétique et d'augmenter la durée de cycle du catalyseur. Pour une réaction en phase liquide, la pression est généralement comprise entre 1 et 3 MPa, la température entre 2 et 50°C et le ratio molaire (hydrogène)/ (composés polyinsaturés à hydrogéner) entre 0,1 et 4, de préférence entre 1 et 2. La V.V.H. est comprise entre 10 h$^{-1}$ et 50 h$^{-1}$.

Pour une réaction d'hydrogénation en phase gazeuse, la pression est généralement comprise entre 1 et 3 MPa, la température entre 40 et 120°C, le ratio molaire (hydrogène)/ (composés polyinsaturés à hydrogéner) entre 0,1 et 4, de préférence entre 1 et 2 et la V.V.H. (débit de charge par volume de catalyseur) est comprise entre 500 h$^{-1}$ et 5000 h$^{-1}$.

Dans le cas d'une hydrogénation sélective d'essence de pyrolyse, le ratio molaire (hydrogène)/(composés polyinsaturés à hydrogéner) est généralement compris entre 1 et 2, la température est généralement comprise entre 40°C et 200°C, de préférence entre 50 et 180°C, la V.V.H. est comprise généralement entre 0,5 h$^{-1}$ et 10 h$^{-1}$, de préférence entre 1 h$^{-1}$ et 5 h$^{-1}$ et la pression est généralement comprise entre 1,0 MPa et 6,5 MPa, de préférence entre 2,0 MPa et 3,5 MPa. Le débit d'hydrogène est ajusté afin d'en disposer en quantité suffisante pour hydrogéner théoriquement l'ensemble des composés polyinsaturés et de maintenir un excès d'hydrogène en sortie de réacteur. Afin de limiter le gradient de température dans le réacteur, il peut être avantageux de recycler une fraction de l'effluent à l'entrée et/ou au milieu du réacteur. L'essence de pyrolyse correspond à une coupe dont la température d'ébullition est généralement comprise entre 0°C et 250°C, de préférence entre 10°C et 220°C. Cette charge comprend généralement la coupe C5-C12 avec des traces de composés en C3, C4, C13, C14, C15 (par exemple entre 0,1 et 3% poids pour chacune de ces coupes). Par exemple, une charge formée d'essence de pyrolyse a généralement une composition en % poids suivante: 8 à 12 % poids de paraffines, 58 à 62 % poids de composés aromatiques, 8 à 10% poids de mono-oléfines, 18 à 22 % poids de dioléfines et de 20 à 300 ppm poids de soufre (partie part million), l'ensemble des composés formant 100%.

L'invention est illustrée par les exemples suivants qui ne présentent, en aucun cas, un caractère limitatif.

## EXEMPLES :

### Exemple 1 : Catalyseur A (non conforme à l'invention)

Une solution aqueuse de nitrate de palladium Pd(NO$_3$)$_2$ est préparée par dilution de 10,88 g d'une solution aqueuse de nitrate de palladium Pd(NO$_3$)$_2$ contenant 10 % poids de nitrate de palladium et 10 % poids d'acide nitrique (Aldrich), avec de l'eau déminéralisée à un volume qui correspond au volume poreux du support. Le pH de cette solution est de 0,74. Par microscopie électronique à transmission, on ne distingue aucune particule d'oxyde de palladium.

Cette solution est ensuite imprégnée sur 100 g d'une alumine dont la surface spécifique est de 140 m$^2$/g et un volume poreux de 1,02 ml/g. Cette alumine est sous forme de billes dont le diamètre moyen est de 3 mm.

Le catalyseur A obtenu est séché sous air à 120°C, puis est calciné pendant 2 heures à 450°C sous un flux d'air avec une V.V.H. de 500 h$^{-1}$. Le catalyseur A contient 0,5% poids Pd par rapport au poids du support.

### Exemple 2 : Catalyseur B (non conforme à l'invention)

Une solution contenant 0,57 g de soude (Prolabo) dans 15 ml d'eau déminéralisée est insérée dans 10,88 g d'une solution aqueuse de nitrate de palladium Pd(NO$_3$)$_2$ contenant 10 % poids de nitrate de palladium et 10 % poids d'acide nitrique (Aldrich). Cette solution est ensuite diluée avec de l'eau déminéralisée à un volume qui correspond au volume poreux du support. Le pH de cette solution est de 1,4. Par microscopie électronique à transmission, on distingue des agglomérats de 20 à 100 nm de nanoparticules d'oxyde de palladium de 1 à 3 nm de diamètre et des nanoparticules d'oxyde de palladium isolées les unes des autres de 1 à 3 nm de diamètre. Moins de 10% des particules se présentent sous forme d'agglomérats.

Cette solution est ensuite imprégnée sur 100 g d'une alumine dont la surface spécifique est de 140 m$^2$/g et un volume poreux de 1,02 ml/g. Cette alumine est sous forme de billes dont le diamètre moyen est de 3 mm.

Le catalyseur B obtenu est séché sous air à 120°C, puis est calciné pendant 2 heures à 150°C, puis 2 heures à 200°C, puis 2 heures à 300°C, puis 2 heures à 450°C sous un flux d'un mélange d'air à 25 vol% et d'azote à 75 vol% avec une V.V.H. de 2000 h$^{-1}$. Le catalyseur B contient 0,5% poids Pd par rapport au poids du support.

Exemple 3 : Catalyseur C (non conforme à l'invention)

0,31 g d'une solution aqueuse à 40 % poids d'hydroxyde de tetrabutylammonium (TBAOH, Aldrich) est ajouté sous agitation à 10,88 g d'une solution aqueuse de nitrate de palladium $Pd(NO_3)_2$ contenant 10 % poids de nitrate de palladium et 10 % poids d'acide nitrique (Aldrich). Cette solution est diluée avec de l'eau déminéralisée à un volume qui correspond au volume poreux du support dans laquelle a été dissout auparavant 1,11 g de soude (Prolabo). Le rapport molaire TBAOH/Pd dans la solution d'imprégnation est égal à 0,1. Le pH de la solution est de 11,2. Par microscopie électronique à transmission, on distingue des agglomérats de 20 à 100 nm de nanoparticules d'oxyde de palladium de 1 à 3 nm de diamètre et des nanoparticules d'oxyde de palladium isolées les unes des autres de 1 à 3 nm de diamètre. Plus de 60% des particules se présentent sous forme d'agglomérats.

Cette solution est ensuite imprégnée sur 100 g d'une alumine dont la surface spécifique est de 140 $m^2$/g et un volume poreux de 1,02 ml/g. Cette alumine est sous forme de billes dont le diamètre moyen est de 3 mm.

Le catalyseur C obtenu est séché sous air à 120°C, puis est calciné pendant 2 heures à 150°C, puis 2 heures à 200°C, puis 2 heures à 300°C, puis 2 heures à 450°C sous un flux d'un mélange d'air à 25 vol% et d'azote à 75 vol% avec une V.V.H. de 2000 $h^{-1}$. Le catalyseur C contient 0,5% poids Pd par rapport au poids du support.

Exemple 4 : Catalyseur D (non conforme à l'invention)

10,88 g d'une solution aqueuse de nitrate de palladium $Pd(NO_3)_2$ contenant 10 % poids de nitrate de palladium et 10 % poids d'acide nitrique (Aldrich) est ajoutée sous agitation à 18,29 g d'une solution aqueuse à 40 % poids d'hydroxyde de tetrabutylammonium (TBAOH, Aldrich). Cette solution est diluée avec de l'eau déminéralisée à un volume qui correspond au volume poreux du support. Le rapport molaire TBAOH/Pd dans la solution d'imprégnation est égal à 6. Le pH de la solution est de 8,2. Par microscopie électronique à transmission, on distingue des agglomérats de 20 à 100 nm de nanoparticules d'oxyde de palladium de 2 à 3 nm de diamètre et des nanoparticules d'oxyde de palladium isolées les unes des autres de 1 à 3 nm de diamètre. Plus de 90% des particules se présentent sous forme d'agglomérats.

Cette solution est ensuite imprégnée sur 100 g d'une alumine dont la surface spécifique est de 140 $m^2$/g et un volume poreux de 1,02 ml/g. Cette alumine est sous forme de billes dont le diamètre moyen est de 3 mm.

Le catalyseur D obtenu est séché sous air à 120°C, puis est calciné pendant 2 heures à 150°C, puis 2 heures à 200°C, puis 2 heures à 300°C, puis 2 heures à 450°C sous un flux d'un mélange d'air à 25 vol% et d'azote à 75 vol% avec une V.V.H. de 2000$h^{-1}$. Le catalyseur D contient 0,5% poids Pd par rapport au poids du support.

Exemple 5 : Catalyseur E (non conforme à l'invention)

2,18 g d'une solution aqueuse de nitrate de palladium $Pd(NO_3)_2$ contenant 10 % poids de nitrate de palladium et 10 % poids d'acide nitrique (Aldrich) est ajoutée sous agitation à 3,66 g d'une solution aqueuse à 40 % poids d'hydroxyde de tetrabutylammonium (TBAOH, Aldrich). Cette solution est diluée avec de l'eau déminéralisée à un volume qui correspond au volume poreux du support. Le rapport molaire TBAOH/Pd dans la solution d'imprégnation est égal à 6. Le pH de cette solution est de 10,1. Par microscopie électronique à transmission, on distingue des agglomérats de 20 à 100 nm de nanoparticules d'oxyde de palladium de 2 à 3 nm de diamètre et des nanoparticules d'oxyde de palladium isolées les unes des autres de 1 à 3 nm de diamètre. Plus de 80% des particules se présentent sous forme d'agglomérats.

Cette solution est ensuite imprégnée sur 100 g d'une alumine dont la surface spécifique est de 140 $m^2$/g et un volume poreux de 1,02 ml/g. Cette alumine est sous forme de billes dont le diamètre moyen est de 3 mm.

Le catalyseur E obtenu est séché sous air à 120°C, puis est calciné pendant 2 heures à 150°C, puis 2 heures à 200°C, puis 2 heures à 300°C, puis 2 heures à 450°C sous un flux d'un mélange d'air à 25 vol% et d'azote à 75 vol% avec une V.V.H. de 2000 $h^{-1}$. Le catalyseur E contient 0,1% poids Pd par rapport au poids du support.

Exemple 6 : Catalyseur F (non conforme à l'invention)

10,88 g d'une solution aqueuse de nitrate de palladium $Pd(NO_3)_2$ contenant 10 % poids de nitrate de palladium et 10 % poids d'acide nitrique (Aldrich) est ajoutée sous agitation à 20,76 g d'une solution aqueuse à 20 % poids d'hydroxyde de tetraethylammonium (TEAOH, Aldrich). Cette solution est diluée avec de l'eau déminéralisée à un volume qui correspond au volume poreux du support. Le rapport molaire TEAOH/Pd dans la solution d'imprégnation est égal à 6. Le pH de cette solution est de 10,6. Par microscopie électronique à transmission, on distingue des agglomérats de 20 à 100 nm de nanoparticules d'oxyde de palladium de 2 à 3 nm de diamètre et des nanoparticules d'oxyde de palladium isolées les unes des autres de 1 à 3 nm de diamètre. Plus de 90 % des particules se présentent sous forme d'agglomérats.

Cette solution est ensuite imprégnée sur 100 g d'une alumine dont la surface spécifique est de 140 $m^2$/g et un volume poreux de 1,02 ml/g. Cette alumine est sous forme de billes dont le diamètre moyen est de 3 mm.

Le catalyseur F obtenu est séché sous air à 120°C, puis est calciné pendant 2 heures à 150°C, puis 2 heures à 200°C, puis 2 heures à 300°C, puis 2 heures à 450°C sous un flux d'un mélange d'air à 25 vol% et d'azote à 75 vol% avec une V.V.H. de 2000 h$^{-1}$. Le catalyseur F contient 0,5% poids Pd par rapport au poids du support.

Exemple 7 : Catalyseur G

Une solution contenant 0,81 g de soude (Prolabo) et 0,68 g de dodecylsulfate de sodium (DS, Aldrich) dans 15 ml d'eau déminéralisée est insérée dans 10,88 g d'une solution aqueuse de nitrate de palladium Pd(NO$_3$)$_2$ contenant 10 % poids de nitrate de palladium et 10 % poids d'acide nitrique (Aldrich). Cette solution est ensuite diluée avec de l'eau déminéralisée à un volume qui correspond au volume poreux du support. Le rapport molaire DS/Pd dans la solution d'imprégnation est égal à 0,5. Le pH de cette solution est de 1,71. Par microscopie électronique à transmission, on distingue des agglomérats de 20 à 100 nm de nanoparticules d'oxyde de palladium de 1 à 3 nm de diamètre et des nanoparticules d'oxyde de palladium isolées les unes des autres de 1 à 3 nm de diamètre. Plus de 70 % des particules se présentent sous forme d'agglomérats.
Cette solution est ensuite imprégnée sur 100 g d'une alumine dont la surface spécifique est de 140 m$^2$/g et un volume poreux de 1,02 ml/g. Cette alumine est sous forme de billes dont le diamètre moyen est de 3 mm.
Le catalyseur G obtenu est séché sous air à 120°C, puis est calciné pendant 2 heures à 150°C, puis 2 heures à 200°C, puis 2 heures à 300°C, puis 2 heures à 450°C sous un flux d'un mélange d'air à 25 vol% et d'azote à 75 vol% avec une V.V.H. de 2000 h$^{-1}$. Le catalyseur G contient 0,5% poids Pd par rapport au poids du support.

Exemple 8 : Catalyseur H

Une solution contenant 0,81 g de soude (Prolabo) et 0,82 g de dodecylbenzenesulfonate de sodium (DBS, Aldrich) dans 15 ml d'eau déminéralisée est insérée dans 10,88 g d'une solution aqueuse de nitrate de palladium Pd(NO$_3$)$_2$ contenant 10 % poids de nitrate de palladium et 10 % poids d'acide nitrique (Aldrich). Cette solution est ensuite diluée avec de l'eau déminéralisée à un volume qui correspond au volume poreux du support d'alumine. Le rapport molaire DBS/Pd dans la solution d'imprégnation est égal à 0,5. Le pH de la solution est de 1,74. Par microscopie électronique à transmission, on distingue des agglomérats de 20 à 100 nm de nanoparticules d'oxyde de palladium de 1 à 3 nm de diamètre et des nanoparticules d'oxyde de palladium isolées les unes des autres de 1 à 3 nm de diamètre. Plus de 70 % des particules se présentent sous forme d'agglomérats.
Cette solution est ensuite imprégnée sur 100 g d'une alumine dont la surface spécifique est de 140 m$^2$/g et un volume poreux de 1,02 ml/g. Cette alumine est sous forme de billes dont le diamètre moyen est de 3 mm.
Le catalyseur H obtenu est séché sous air à 120°C, puis est calciné pendant 2 heures à 150°C, puis 2 heures à 200°C, puis 2 heures à 300°C, puis 2 heures à 450°C sous un flux d'un mélange d'air à 25 vol% et d'azote à 75 vol% avec une V.V.H. de 2000h$^{-1}$. Le catalyseur H contient 0,5 % poids Pd par rapport au poids du support.

Exemple 9 : Test catalytique en hydrogénation d'un mélange styrène isoprène

Avant le test catalytique, les catalyseurs A, B, C, D, E, F, G et H sont traités sous un flux d'hydrogène avec une V.V.H. de 500 h$^{-1}$ avec une montée en température de 300°C/h et un palier à une température finale de 150°C pendant 2 heures.
Les catalyseurs sont ensuite soumis à un test d'hydrogénation dans un réacteur discontinu parfaitement agité de type « Grignard ». Pour ce faire, 4 ml de billes de catalyseur réduit sont fixées à l'abri de l'air dans un panier annulaire situé autour du mobile d'agitation. Les paniers utilisés dans les réacteurs sont de type Robinson Mahonnay.
L'hydrogénation est réalisée en phase liquide.
La composition de la charge est la suivante : 8% poids styrène, 8% poids isoprène, 74% de n-heptane.
Le test est réalisé sous une pression constante de 3,5 MPa d'hydrogène et à une température de 45 °C.
Les produits de la réaction sont analysés par chromatographie en phase gazeuse.
Les activités catalytiques sont exprimées en moles de H$_2$ consommées par minute et par gramme de palladium et sont reportées dans le tableau 1.

Tableau 1: Activités mesurées en hydrogénation d'un mélange styrène-isoprène

| Catalyseur | Activité* |
|---|---|
| Catalyseur A (non conforme) | 1,84 |
| Catalyseur B (non conforme) | 2,73 |
| Catalyseur C (non conforme) | 4,29 |

(suite)

| Catalyseur | Activité* |
|---|---|
| Catalyseur D (non conforme) | 6,43 |
| Catalyseur E (non conforme) | 10,70 |
| Catalyseur F (non conforme) | 6,82 |
| Catalyseur G | 6,38 |
| Catalyseur H | 5,89 |
| * en (moles H$_2$)/[min$\times$(gramme de palladium)] | |

Les catalyseurs G et H conformes à l'invention sont de environ 1,5 à 6 fois plus actifs que les catalyseurs A et B non conformes à l'invention.

Exemple 10 : Répartition de la phase métallique sur le support

Afin d'analyser la répartition de la phase métallique sur le support, on mesure une épaisseur de croûte par microsonde de Castaing (ou microanalyse par microsonde électronique). L'appareil utilisé est un CAMECA XS100, équipé de quatre cristaux monochromateurs permettant l'analyse simultanée de quatre éléments. La technique d'analyse par microsonde de Castaing consiste en la détection de rayonnement X émis par un solide après excitation de ses éléments par un faisceau d'électrons de hautes énergies. Pour les besoins de cette caractérisation, les billes de catalyseur sont enrobées dans des plots de résine époxy. Ces plots sont polis jusqu'à atteindre la coupe au diamètre des billes puis métallisés par dépôt de carbone en évaporateur métallique. La sonde électronique est balayée le long du diamètre de cinq billes pour obtenir le profil de répartition moyen des éléments constitutifs des solides.

Pour mesurer une épaisseur de croûte qui est significative pour la majorité des particules de palladium, l'épaisseur de croûte peut de façon alternative être définie comme la distance au bord du grain contenant 80% en poids du palladium. A partir du profil de répartition obtenu par la microsonde de Castaing ("c(x)"), on peut calculer la quantité cumulée de Pd dans le grain en fonction de la distance "y" au bord du grain de rayon "r". Pour une bille :

$$Q(y) = \int_{-r}^{-y} c(x).4.\pi.x^2.dx + \int_{y}^{r} c(x).4.\pi.x^2.dx$$

$Q(r)$ correspondant ainsi à la quantité totale de Pd dans le grain. On résout ensuite numériquement l'équation suivante en y afin d'obtenir l'épaisseur de croûte à 80% poids en palladium :

$$\frac{Q(y)}{Q(r)} = 0.8$$

Les épaisseurs de croûte des différents catalyseurs sont reportées au tableau 2.

**Tableau 2 : Épaisseurs de croûte métallique mesurées par microsonde de Castaing**

| Catalyseur | Épaisseur de croûte (en $\mu$m) comprenant 80% poids Pd* |
|---|---|
| Catalyseur A (non conforme) | 125 |
| Catalyseur B (non conforme) | 73 |
| Catalyseur C | 54 |
| Catalyseur D | 36 |
| Catalyseur E | 21 |
| Catalyseur F | 35 |
| Catalyseur G | 38 |

EP 2 474 354 B1

(suite)

| Catalyseur | Épaisseur de croûte (en $\mu$m) comprenant 80% poids Pd* |
|---|---|
| Catalyseur H | 43 |

**Revendications**

1. Procédé de préparation d'un catalyseur comprenant les étapes suivantes:

    (a) on prépare une suspension aqueuse colloïdale d'agglomérats de nanoparticules d'oxyde métallique, ladite suspension aqueuse colloïdale étant obtenue par hydrolyse en solution aqueuse, en absence d'agent réducteur et en absence de solvant organique, d'au moins un sel d'un précurseur métallique et en présence d'au moins un agent agglomérant, ledit métal dudit sel de précurseur métallique étant choisi dans le groupe constitué par le palladium et le nickel, ledit agent agglomérant étant un agent agglomérant anionique choisi dans le groupe formé par le dodécylsulfate de sodium et le dodécylbenzènesulfonate de sodium, le rapport molaire entre l'agent agglomérant et le sel d'un précurseur métallique est compris entre 0,001 et 100, la préparation de ladite suspension colloïdale s'effectuant à une température comprise entre 10 et 50°C et dans des conditions normales de pression (pression atmosphérique), le pH de ladite solution colloïdale étant inférieur au point de charge nulle de l'oxyde de métal ;
    la suspension ainsi préparée est telle qu'entre 20 % et 95 % en nombre des nanoparticules d'oxyde métallique se trouvent sous forme d'agglomérats, lesdits agglomérats ayant une taille comprise entre 20 et 200 nm mesurée par microscopie électronique à transmission (MET),
    (b) on dépose ladite suspension aqueuse colloïdale d'agglomérats obtenue à l'étape (a) sur un support poreux,
    (c) on sèche le produit obtenu à l'étape (b).

2. Procédé de préparation selon la revendication 1 comprenant en outre après l'étape (c), au moins une étape (d) de calcination du produit obtenu à l'étape (c).

3. Procédé de préparation selon l'une des revendications 1 ou 2 comprenant en outre, et après l'une des étapes (c) ou (d), au moins une étape de réduction du produit obtenu à l'étape (c) ou (d).

4. Procédé de préparation selon l'une quelconque des revendications 1 à 3, dans lequel ledit sel d'un précurseur métallique est choisi dans le groupe constitué par un halogénure, un oxyde, un hydroxyde, un nitrate et un sulfate du métal.

5. Procédé de préparation selon l'une quelconque des revendications 1 à 4, dans lequel le rapport molaire entre l'agent agglomérant et le sel d'un précurseur métallique est compris entre 0,001 et 100; de préférence entre 0,01 et 50 et de manière encore plus préférée entre 0,02 et 10.

6. Procédé de préparation selon l'une quelconque des revendications 1 à 5 dans lequel l'hydrolyse est réalisée par neutralisation avec au moins une base minérale.

**Patentansprüche**

1. Verfahren zur Herstellung eines Katalysators, umfassend die folgenden Schritte:

    (a) Herstellen einer kolloidalen wässrigen Suspension von Agglomeraten von Nanopartikeln eines Metalloxids, wobei die kolloidale wässrige Suspension durch Hydrolyse in wässriger Lösung, ohne Reduktionsmittel und ohne organisches Lösungsmittel, mindestens eines Salzes einer Metallvorstufe und im Beisein mindestens eines Agglomerationsmittels erhalten wird, wobei das Metall des Salzes einer Metallvorstufe in der Gruppe ausgewählt ist, die von Palladium und Nickel gebildet ist, wobei das Agglomerationsmittel ein anionisches Agglomerationsmittel ist, das in der Gruppe ausgewählt ist, die von Natriumdodecylsulfat und Natriumdodecylbenzolsulfonat gebildet ist, wobei das Molverhältnis zwischen dem Agglomerationsmittel und dem Salz einer Metallvorstufe zwischen 0,001 und 100 beträgt, wobei die Herstellung der kolloidalen Suspension bei einer Temperatur zwischen 10 und 50 °C und unter normalen Druckbedingungen (Atmosphärendruck) erfolgt, wobei

**11**

der pH-Wert der kolloidalen Lösung unter dem Ladungsnullpunkt des Metalloxids liegt;
wobei die so hergestellte Suspension derart ist, dass zwischen 20 % und 95 % in Anzahl der Nanopartikel von Metalloxiden in Form von Agglomeraten vorhanden sind, wobei die Agglomerate eine Größe zwischen 20 und 200 nm, gemessen durch Transmissionselektronenmikroskopie (MET), haben,
(b) Aufbringen der in Schritt (a) erhaltenen kolloidalen wässrigen Suspension von Agglomeraten auf einen porösen Träger,
(c) Trocknen des in Schritt (b) erhaltenen Produkts.

2. Herstellungsverfahren nach Anspruch 1, ferner umfassend nach dem Schritt (c) mindestens einen Schritt (d) des Kalzinierens des in Schritt (c) erhaltenen Produkts.

3. Herstellungsverfahren nach einem der Ansprüche 1 oder 2, ferner umfassend nach einem der Schritte (c) oder (d) mindestens einen Reduktionsschritt des in Schritt (c) oder (d) erhaltenen Produkts.

4. Herstellungsverfahren nach einem der Ansprüche 1 bis 3, bei dem das Salz einer metallischen Vorstufe in der Gruppe ausgewählt ist, die von einem Halogenid, einem Oxid, einem Hydroxid, einem Nitrat und einem Sulfat des Metalls gebildet ist.

5. Herstellungsverfahren nach einem der Ansprüche 1 bis 4, bei dem das Molverhältnis zwischen dem Agglomerationsmittel und dem Salz einer metallischen Vorstufe zwischen 0,001 und 100; vorzugsweise zwischen 0,01 und 50 und noch bevorzugter zwischen 0,02 und 10 liegt.

6. Herstellungsverfahren nach einem der Ansprüche 1 bis 5, bei dem die Hydrolyse durch Neutralisierung mit mindestens einer mineralischen Basis erfolgt.

**Claims**

1. A process for the preparation of a catalyst comprising the following steps:

(a) preparing a colloidal aqueous suspension of agglomerates of nanoparticles of metallic oxide, said colloidal aqueous suspension being obtained by hydrolysis in aqueous solution, in the absence of a reducing agent and in the absence of an organic solvent, of at least one salt of a metallic precursor and in the presence of at least one agglomerating agent, said metal of said salt of the metallic precursor being selected from the group formed by palladium and nickel, said agglomerating agent being an anionic agglomerating agent selected from the group formed by sodium dodecyl sulphate and sodium dodecylbenzene sulphonate, the molar ratio between the agglomerating agent and the salt of a metallic precursor is between 0.001 and 100, the preparation of said colloidal suspension is effected at a temperature between 10 and 50°C under normal pressure conditions (atmospheric pressure), pH of the colloidal solution is lower than the zero charge point of the metal oxide;
the colloidal suspension of agglomerate, that is prepared in that way, is such that between 20% and 95% by number of the nanoparticles of metal oxide are in the form of agglomerates, the size of said agglomerates is between 20 and 200 nm measured by transmission electron microscopy (TEM),
(b) depositing said colloidal aqueous suspension of agglomerates obtained in step (a) on a porous support, and
(c) drying the product obtained in step (b).

2. A preparation process according to claim 1 further comprising after step (c) at least a step (d) for calcination of the product obtained in step (c).

3. A preparation process according to one of claims 1 or 2 further comprising and after one of steps (c) or (d) at least a step for reduction of the product obtained in step (c) or (d).

4. A preparation process according to one of claims 1 to 3 in which said salt of a metallic precursor is selected from the group formed by a halide, an oxide, a hydroxide, a nitrate and a sulphate of the metal.

5. A preparation process according to any one of claims 1 to 4 wherein the molar ratio between the agglomerating agent and the salt of a metallic precursor is between 0.001 and 100; preferably between 0.01 and 50 and still more preferably between 0.02 and 10.

6. A preparation process according to any one of claims 1 to 5 wherein the hydrolysis operation is effected by neutralisation with at least one inorganic acid.

# RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 0979673 A
- WO 0029332 A
- EP 0653243 A

**Littérature non-brevet citée dans la description**

- **M. BOUDART ; W.C. CHENG.** *J. Catal.,* 1987, vol. 106, 134
- **S. HUB ; L. HILAIRE ; R. TOUROUDE.** *Appl. Catal.,* 1992, vol. 36, 307
- **KLASOVSKY et al.** *Topic in Catalysis,* 2009, vol. 52, 412-423
- **D.R. LIDE.** CRC Handbook of Chemistry and Physics. 2000